# EUROPEAN PATENT SPECIFICATION

(11) **EP 3 495 510 B1**
(45) Date of publication and mention of the grant of the patent: **21.07.2021**
(21) Application number: 18210901.7
(22) Date of filing: 06.12.2018
(51) Int. Cl.: C12R 1/01, C12R 1/425, A01N 63/00, C12N 1/00

(54) **ANTAGONISTIC BACTERIAL STRAINS, COMPOSITIONS THEREOF AND USE FOR PLANT PROTECTION**
ANTAGONISTISCHE BAKTERIENSTÄMME, ZUSAMMENSETZUNGEN HIERVON UND VERWENDUNG IM PFLANZENSCHUTZ
SOUCHES BACTÉRIENNES ANTAGONISTES, COMPOSÉS LES COMPRENANT ET UTILISATION POUR LA PROTECTION DES PLANTES

(30) Priority: 08.12.2017 PL 42380617
(43) Date of publication of application: 12.06.2019
(73) Proprietor: Uniwersytet Gdanski, 80-309 Gdansk (PL)
(72) Inventor: Czajkowski, Robert, 80-382 Gdansk (PL); Krzyzanowska, Dorota, 81-572 Gdynia (PL); Maciag, Tomasz, 85-337 Bydgoszcz (PL); Jafra, Sylwia, 80-382 Gdansk (PL); Siwinska, Joanna, 80-177 Gdansk (PL)
(74) Representative: Pawlowska, Justyna

(56) References cited:
- WO-A1-2012/095431
- R. CZAJKOWSKI ET AL: "Studies on the interaction between the biocontrol agent, Serratia plymuthica A30, and blackleg-causing Dickeya sp. (biovar 3) in potato (Solanum tuberosum)", PLANT PATHOLOGY, 1 November 2011 (2011-11-01), pages no-no, XP055021833, ISSN: 0032-0862, DOI: 10.1111/j.1365-3059.2011.02565.x
- JAFRA S ET AL: "Potential of bulb-associated bacteria for biocontrol of hyacinth soft rot caused by Dickeya zeae", JOURNAL OF APPLIED MICROBIOLOGY, WILEY-BLACKWELL PUBLISHING LTD, GB , vol. 106, no. 1 1 January 2009 (2009-01-01), pages 268-277, XP002671554, ISSN: 1364-5072, DOI: 10.1111/J.1365-2672.2008.04000.X Retrieved from the Internet: URL:http://onlinelibrary.wiley.com/doi/10. 1111/j.1365-2672.2008.04000.x/abstract [retrieved on 2008-12-01]
- CZAJKOWSKI R ET AL: "Characterization of bacterial isolates from rotting potato tuber tissue showing antagonism to Dickeya sp. biovar 3 in vitro and in planta", PLANT PATHOLOGY, WILEY-BLACKWELL PUBLISHING LTD, GB , vol. 61, no. 1 1 February 2012 (2012-02-01), pages 169-182, XP002671555, ISSN: 0032-0862, DOI: 10.1111/J.1365-3059.2011.02486.X Retrieved from the Internet: URL:http://onlinelibrary.wiley.com/doi/10. 1111/j.1365-3059.2011.02486.x/abstract [retrieved on 2011-06-20]

## Description

The invention relates to new strains of antagonistic bacteria in composition as well as its use as a natural pesticides for protecting healthy plants against bacterial infections and/or use of the strain for treating plants showing disease symptoms caused by bacterial infection. In particular, the invention relates to the protection and treatment of plant diseases caused by pectinolytic bacteria, especially the genera *Pectobacterium* and *Dickeya,* and according to the classification in force in December 2018: bacteria of the genus *Erwinia*, pectinolytic bacteria of the genus *Erwinia,* pectinolytic bacteria of the family *Enterobacteriaceae,* bacteria of the family *Enterobacteriaceae* causing soft rot, pectinolytic bacteria of the family *Pectobacteriaceae,* bacteria of the family *Pectobacteriaceae* causing soft rot, bacteria of the family *Pectobacteriaceae,* also bacteria belonging to the genera *Ralstonia* and *Clavibacter,* which cause diseases in plants especially such as soft rot, blackleg, brown rot, ring rot.

Arable crops are susceptible to infection by pathogens, which on a global scale leads to enormous economic losses in the production of food, animal feed and in industry. Methods used to protect crops against pathogens include, among others, applying chemical plant protection products, preventing the transfer and propagation of bacteria from infected and symptomatic plants to healthy ones and inspection of agricultural fields to determine whether plant material is free from pathogens.

The commonly used chemical methods of plant protection (e.g. chemical pesticides) against pathogens are not only insufficiently effective for plant protection, but above all their use leads to increasing pollution of environmental, dangerous to human and animal health, as well as to degradation of natural soil microbiota. It is worth emphasizing that plant pathogens can quickly become resistant to chemical pesticides, which further justifies the need to search for new, alternative methods of protecting plants against plant pathogens.

Biological protection of plants (biological control of plant pathogens) involves the use of the organism or organisms antagonistic to the pathogen, which cause a reduction in pathogen's number, inhibition of the development of disease symptoms caused by the pathogen or the elimination of the pathogen. The mechanism of biological control may rely on various mechanisms, comprising, among others, the production of antibiotics, competition for environmental resources (e.g. competition for iron ions - production of siderophores), parasitizing of the pathogenic organism, or induction of a defence response in the plant (e.g. by producing phytohormones and phytostimulants).

The estimated global market value of agricultural pesticides is over 30 billion USD, of which biological plant protection agents (biopesticides) comprise only about 1%. Almost all biopesticides used in agriculture are based on the use of only a few species / genera of bacteria (e.g. *Bacillus* spp., *Pseudomonas* spp. *Pantoea* spp., *Agrobacterium radiobacter*)*.* In Europe, at the beginning of the XXI century, the value of biological plant protection products amounted to approximately 100 million USD, of which 20 million USD was attributed to products based on microorganisms. Sobiczewski and co-authors reported that in 2009 in Europe only 35 companies produced and sold microbiological agents for plant protection. There is a huge demand for environmentally friendly microbiological formulations, such as microbiological agents, necessary to reduce chemical protection and to ensure a constant quality and quantity of crops. Environmental awareness of consumers of agricultural products is constantly increasing, accompanied by an increase in the area of organic farming. It is assumed that this trend will continue.

Antagonistic bacteria are bacteria that live on plant tissues or within them (endophytes) or in the vicinity of roots (rhizosphere) and on the surface of leaves (phyllosphere). These bacteria can actively protect the plant by supporting the uptake of minerals from the soil, inhibiting the development of pathogenic bacteria and by the production of phytohormones stimulating plant growth and health. Antagonistic bacteria interact, directly and indirectly, with many (pathogenic) organisms existing in / on plants and in their vicinity, and therefore, play the role of biological plant protection.

The use of biological plant protection based on antagonistic bacteria is an attractive alternative to traditional plant protection because this approach is based on natural antagonisms, and not on the action of chemicals, often toxic to humans and animals. The use of antagonistic bacteria does not introduce any foreign factors into the natural environment, which could be toxic to humans and animals and that may accumulate at the subsequent levels of the ecological pyramid.

Moreover, most of the currently used commercial products for biological plant protection is in the form of living cells of bacteria or their spores, which, as naturally occurring in the soil environment, do not pose a threat. Biological protection may be applied during the growing season or during the harvesting and storage of crops, while maintaining the safety of the latter for the natural environment, humans and animals.

Pectinolytic bacteria cause disease symptoms on economically important arable crops such as, among others: potato, tomato, carrot, onion, garlic, cucumber, peppers and ornamental plants such as, among others: cyclamen, hyacinth, tulip, orchid and chrysanthemum.

In the examples confirming the positive effect of use of the antagonistic bacteria according to the present invention, potato tubers were used as the model plant system. Potato (*Solanum tuberosum* L.) is one of the most important arable crops, both due to the area of cultivation and the total production of potato tubers. In terms of importance for the world's agriculture, it is the fourth staple crop after rice, maize and wheat. The intensive cultivation and production of potato in Europe (crop monocultures) as well as the international trade in plant material contributes to an increased risk of spreading diseases. All potato diseases contribute to high losses in yield quantity and quality. Diseases cause losses at every stage of potato cultivation / production and relate to roots, aerial parts of stems, and above all, and most often, potato tubers.

An important problem in the cultivation / storage of plants are infections caused by pectinolytic bacteria, especially of the genera *Pectobacterium* and *Dickeya,* or their equivalents depending on the current taxonomic classification. These bacteria most often cause soft rot during storage and transit, especially on such plants as potatoes, tomatoes, carrots, onions, garlic, cucumbers, peppers and ornamental plants such as cyclamen, hyacinth, tulip, orchid, chrysanthemum, as well as rotting of potato stems (the so-called blackleg) during the growing season.

In the case of potato infections, it is generally accepted that the main source of infection of new plants with the pectinolytic bacteria of the genera *Pectobacterium* and *Dickeya* are latently (asymptomatically) infected seed tubers, which, during plant growth in the field, release bacteria into the environment and spread infection to new plants, progeny tubers, other crops located in the same or neighbouring fields, and between crops and fields. Therefore, the production of pathogen-free seed potatoes is the most important strategy in reducing the possibility of infection of healthy plants with pectinolytic bacteria.

Typical soft rot symptoms on tubers are: rot which begins in the buds, at the ends of the stolons, or in wounds formed during harvesting of tubers. The presence of bacteria leads to the maceration of potato tissues, which, when rotting, take on a creamy colour and a watery consistency, blacken in the air and become infected by other (opportunistic) microorganisms that can cause secondary disease symptoms.

One infected tuber during storage, especially under conditions of insufficient temperature and humidity control (high temperature, high humidity), may cause infection of up to a hundred of the neighbouring (healthy) tubers adjacent to the infected one.

For example, the measures to control the pathogens of the genera *Pectobacterium* and *Dickeya* on potato crops are limited to:
1. the use of seed material originating from *in vitro* potato cultivation, free from pathogens
2. field inspections and occasional laboratory tests carried out to determine if the seed material is free from pathogens
3. application of preventive procedures: disinfection of machines used in the field, facilities for storage of tubers, as well as prevention of the transfer of the bacterial inoculum onto healthy plants during the entire production period.

In the case of potato, none of the above methods of control, nor a combination thereof, allows for 100% protection of plants and potato tubers against infection, but only contributes to a partial reduction of crop losses. Methods of controlling pectinolytic bacteria on potato are insufficient, which is observed especially in the years in which the weather during the growing season of potato (from April to September) encourages the development of disease symptoms (high rainfall, high temperature).

One of the key problems with pectinolytic bacteria is that in the course of infection they reside in niches that are difficult to access with antibacterial agents. Pectinolytic bacteria reside inside tubers and stems, while chemical or physical protection agents act on the plant surface and do not penetrate inside the tissues.

There are no effective systemic plant protection agents that can infiltrate crop plants through roots or leaves and spread within the plant tissues, providing protection. Therefore, there is a constant need to improve available plant protection products and to develop new ones. All methods currently used to protect seed material (including seed potatoes) against pectinolytic bacteria can at most contribute to reducing the level of infection, but are unable to completely eliminate bacteria from infected plants and to ensure protection of healthy plants.

This results from the inability for chemical compounds and physical factors to penetrate inside the plants and to access the locations where the highest populations of the pathogen are present during the infection. Bacteria of the genera *Pectobacterium and Dickeya* are pathogens inhabiting the vascular tissues of plants, while the antibacterial chemicals used in agriculture act only on the surface and do not penetrate inside these tissues. Similarly, physical factors such as UV radiation and solar radiation cannot penetrate inside the tubers and stems. Gamma radiation, which penetrates into the tissues, and incubation in hot water, which heats tuber inner tissues, both promote the damage of seed potatoes (lack or limitation of crop emergence in the field), and hence, are rarely used in practice.

The only method of partial control of pectinolytic bacterial infection is prevention, involving the control of the spread of the infection in the field through cleaning of the mechanical equipment used for harvesting with chemical agents, the use of more resistant plant varieties and the elimination of infected plants during growth. In practice, farmers may also use plant protection products applied when planting tubers in the field or applied during plant growth in the growing season.

Estimated losses in potato production in Europe caused by pectinolytic bacteria are high (loss of about 10% - 20% yields) and currently amount to around 250 million EUR a year. For this reason, the bacteria of the *Pectobacterium* spp. and *Dickeya* spp. are among the top five bacterial plant pathogens in terms of generated losses. Therefore, it is reasonable to use protection agents based on microorganisms that are both human and environmentally friendly and competitive in relation to the methods currently used.

From US5552315, there are known antagonistic bacteria that are useful in controlling potato disease caused by fungi. Similarly, in the description US 8623390 A, antagonistic bacteria have been disclosed for use in potato diseases. In another description, US 8278246 A, compositions of antagonistic bacteria are disclosed, aimed at inhibiting potato diseases caused by fungi.

The solutions regarding individual bacterial strains suitable for treating or preventing infections with pectinolytic bacteria are known. In particular, in WO2012095431 Serratia plymuthica strain A30 provides a biological control agent against plant disease caused by a bacteria pathogen, particularly a soft rot, blackleg. There is description about pathogens including *Dickeya spp., Pectobacterium spp.,* and *Ralstonia spp.;* including *Dickeya spp. Biovar 3* - *Dickeya solani*)..

In Czajkowski et al.: studies on the interaction between the biocontrol agent, Serratia plymuthica A30, and blackleg-causing Dickeya sp. (biovar 3) in potato (Solanum tuberosum) , Plant Pathology 2011, interactions between *Serratia plymuthica* A30 and a blackleg-causing biovar 3 *Dickeya sp. - D. solani* were dislosed. In Jafra et. al.: potential of bulb-associated bacteria for biocontrol of hyacinth soft rot caused by Dickeya zeae, Journal of Applied Microbiology 2009, bacterial isolates with potential for biocontrol were selected on the basis of antibiosis against *D. zeae* on hyacinth bulbs: *Rahnella aquatilis* or *Erwinia persicinus* were disclosed. In Czajkowski et. al: characterization of bacterial isolates from rotting potato tuber tissue showing antagonism to *Dickeya sp. biovar* 3 - *D. solani* in vitro and in planta, Plant Pathology 2011, the potential use of *S. plymuthica A30* for the biocontrol of *Dickeya sp.* was discussed.

None of the mentioned documents describe the use either of a mixture of antagonistic strains or the use of antagonistic strains against a mixture of pathogens.

The object of the present invention is to provide an effective method of protecting plants against infection caused by phytopathogens - pectinolytic bacteria, especially of the genera *Pectobacterium* and *Dickeya* or their equivalents. Unexpectedly, this problem has been substantially solved in the present invention using unique antagonistic strains in mixture thereof.

The invention relates to the new strains listed below in the composition - a mixture of five strains:
- the strain of the antagonistic bacteria *Serratia plymuthica,* identification reference: strain A294, being deposited on 1 December 2017 with the Polish Collection of Microorganisms PCM at the Ludwik Hirszfeld Institute of Immunology and Experimental Therapy in Wroclaw, Polish Academy of Sciences, under accession number B/00143;
- the strain of the antagonistic bacteria *Serratia rubidaea*, identification reference: strain H469, being deposited on 1 December 2017 with the Polish Collection of Microorganisms PCM at the Ludwik Hirszfeld Institute of Immunology and Experimental Therapy in Wroclaw, Polish Academy of Sciences, accession under number B/00142;
- the strain of the antagonistic bacteria *Serratia rubidaea*, identification reference: strain H440, being deposited on 1 December 2017 with the Polish Collection of Microorganisms PCM at the Ludwik Hirszfeld Institute of Immunology and Experimental Therapy in Wroclaw, Polish Academy of Sciences, under number B/00141;
- the strain of the antagonistic bacteria *Enterobacter amnigenus,* identification reference: strain A167, being deposited on 1 December 2017 with the Polish Collection of Microorganisms PCM at the Ludwik Hirszfeld Institute of Immunology and Experimental Therapy in Wroclaw, Polish Academy of Sciences, accession under number B/00145;
- the strain of the antagonistic bacteria *Rahnella aquatilis*, identification reference: strain H145, being deposited on 1 December 2017 with the Polish Collection of Microorganisms PCM at the Ludwik Hirszfeld Institute of Immunology and Experimental Therapy in Wroclaw, Polish Academy of Sciences under accession number B/00144.

Preferably, the strains in the mixture of 5 strains - the composition - are present in an equal ratio of each of the strain in comparison to the other strains or one another. The number of cells of each strain in the mixture can be at least 10¹ cells in millilitre (CFU / ml), preferably 10⁸ CFU / ml. The mixture is in lyophilized form or in a suspension. The strains may be metabolically active.

The use of an antagonistic bacterial strains in the composition according to the invention includes the prevention or treatment of plant infections caused by pectinolytic bacteria, particularly infections causing the soft rot of vegetables or blackleg diseases of potato. The plants in which the strain can be used are, among others: potato, carrot, tomato, onion, pepper, garlic, cucumber, cyclamen, hyacinth, tulip, orchid or chrysanthemum. Use of the composition is preferably for prevention or treatment of potato infections caused by pectinolytic bacteria *Dickeya spp.* and/or *Pectobacterium spp.*

The invention is applicable - used - under plant storage conditions, field conditions, hydroponic conditions, in greenhouse crops, and/or *in vitro* grown plants. The bacteria, either alone or in a mixture - composition, are applied by vacuum infiltration, and / or spraying and / or fogging and / or sprinkling the plants and / or drenching the plants under storage conditions, in greenhouse, field, under hydroponic conditions, where antagonistic bacteria inhibit the disease symptoms caused by pectinolytic bacteria in the range of temperatures from 7°C to 40°C. The area of application is the surface of the plant, compost, soil, sand or other medium in which plants may be grown under field conditions, in greenhouses and in *in vitro* cultures.

Regarding similarity between bacterial strains in the composition, according to the invention, in microbiology, a species is essentially defined as a collection of strains that are characterized by at least one diagnostic phenotype and whose purified genomic DNA display at least 70% cross-hybridization (DNA-DNA hybridization). There are also other methods for assigning strains to a given species, providing different resolution, including those based on the degree of identity between 16S rRNA gene sequences. Importantly, individual strains from a given species can have very different properties even if the genetic differences between these strains are very small in relation to total genetic information encoded in the genomes of these microorganisms. An example of significant differences in the properties of bacteria from the perspective of their importance to humans are bacteria of the *Escherichia coli* species. The *E. coli* bacteria are an important element of physiological flora of healthy individuals. Meanwhile, some strains of this species, due to the presence of a gene encoding enterotoxin, can cause serious disease symptoms in humans. Similarly, in the case of species which include strains of bacteria used in biological plant protection, not every representative of a given species will exhibit the desired protective properties or other beneficial characteristics, e.g. those determining the compatibility of given strains with other, simultaneously applied protective microorganisms.

Despite this, there are common features between the strains according to the invention.

The strains according to the inventionin a mixture, exhibit antagonism against pectinolytic bacteria of the genus *Pectobacterium* and *Dickeya,* or their equivalents according to changes in classification, and the basis of this observed antagonism is the production of various factors affecting the growth of pectinolytic *Pectobacterium* and *Dickeya* bacteria. The comparison between effect of use of the strain individually and in the composition according to the invention is presented in the examples. Specifically, the *Serratia plymuthica* strain A294 is a Gram-negative bacterium that produces plant hormones that have a positive effect on plant development and produces antibiotics against bacteria of the genera *Pectobacterium* and *Dickeya.* The *Enterobacter amnigenus* strain A167 is a Gram-negative bacterium, producing agents that interfere with the communication of bacteria of the genera *Pectobacterium* and *Dickeya* by degrading signal molecules (the so called quorum-sensing-interfering - quorum quenching). The *Rahnella aquatilis* strain H145 is a Gram-negative bacterium producing siderophores chelating iron ions and producing antibiotics against bacteria of the genera *Pectobacterium* and *Dickeya.* The *Serratia rubidaea* strain H440 is a Gram-negative bacterium producing siderophores chelating iron ions from the environment and producing agents interfering with the communication of bacteria of the genera *Pectobacterium* and *Dickeya* in the environment, by degrading signal molecules (the so called quorum-sensing-interfering - quorum quenching). The *Serratia rubidaea* strain H469 is a Gram-negative bacterium producing siderophores chelating iron ions from the environment and producing antibiotics against bacteria of the genera *Pectobacterium* and *Dickeya.* All isolates of antagonistic bacteria are plant-associated bacteria and have been isolated from the vicinity of plants.

The presented invention confirms that it is a big advantage in biological control methods (biological methods of pathogen control - natural methods) being used to reduce disease symptoms caused by pectinolytic bacteria, especially pathogenic bacteria of the genera *Pectobacterium* and *Dickeya,* as evidenced here by the example of potato tuber protection. From the perspective of the application, it is important that selected and tested antagonistic bacterial strains are naturally present on plants, in their tissues (as endophytes) or in a root zone (rhizosphere) and on the leaf surface (phyllosphere), therefore it is their typical living environment, which will additionally have a positive effect on the length of their survival and the stability of their populations after application.

The antagonistic bacterial strains according to the invention, used in the composition, such as: the *Serratia plymuthica* strain A294 and the *Enterobacter amnigenus* strain A167 and the *Rahnella aquatilis* strain H145 and the *Serratia rubidaea* strains H440 and H469 sets an effective product showing antagonism against pectinolytic bacteria of the genera *Pectobacterium* and *Dickeya,* or their equivalents. The mixture of 5 bacterial strains is particularly effective when the components of the mixture are mixed together in substantially equal proportions. The invention is effective against single species of pectinolytic bacteria and is effective against a mixture of different species of pectinolytic bacteria.

Inventors of said invention, with the example of potato tuber tissue, also proved that the use of the mixture of 5 antagonistic bacteria protects against the occurrence of disease symptoms caused by *Pectobacterium* and *Dickeya* in plants. The inventors, with the example of potato tissues, showed that the antagonistic bacteria according to the invention used in the composition are able to survive under a wide range of temperature conditions and are able to survive on the surface of plants. Other plants on which the mixture of the invention may be used, apart from potato, are, e.g. carrot, tomato, onion, pepper, garlic, cucumber, cyclamen, hyacinth, tulip, orchid and/or chrysanthemum. The mixture of 5 strains can also be used on other plants if the pathogens, as to which the mixture is active, cause disease symptoms on these plants. The invention is applicable - used: under plant storage conditions, field conditions, hydroponic conditions, on greenhouse crops, on *in vitro* crops. The invention can be applied by vacuum infiltration, spraying, fogging, sprinkling of plants, dipping plants under storage conditions, in greenhouse, field, and under hydroponic conditions. The target area for application of the mixture is the plant surface, compost, soil, sand or other medium in which the plants can be grown under field conditions, in greenhouses and in *in vitro* cultures.

The terms used in this patent application have the following meanings:
Antagonistic interactions - a kind of interspecies interaction between different bacterial populations, considered unfavourable for one of the populations occupying the same environment.
Antagonistic bacteria - bacteria with antagonistic potential against bacteria of the genera *Pectobacterium* and *Dickeya*, synonym: protective bacteria.
Pectinolytic bacteria - bacteria of the genera *Pectobacterium* and *Dickeya,* synonym: pectinolytic bacteria of the family *Enterobacteriaceae,* resulting in the occurrence of disease symptoms in plants, including potato plants, causing blackleg and soft rot, synonym: pathogenic bacteria. According to the classification in force in December 2018: bacteria of the genus *Erwinia,* pectinolytic bacteria of the genus *Erwinia,* pectinolytic bacteria of the family *Enterobacteriaceae,* bacteria of the family *Enterobacteriaceae* causing soft rot, pectinolytic bacteria of the family *Pectobacteriaceae,* bacteria of the family *Pectobacteriaceae* causing soft rot, bacteria of the family *Pectobacteriaceae,* also bacteria belonging to the genera *Ralstonia* and *Clavibacter,* which cause diseases in plants such as, especially, soft rot, blackleg, brown rot, ring rot.
CFU - interchangeably "colony forming units" a unit expressing the number of viable bacterial cells, per ml of suspension (CFU/ml).
Mixture - for the purposes of the examples, interchangeably it also means "composition" of at least two bacterial strains.
V/V - means the volume / volume ratio.
GF - a five-component mixture of protective bacteria containing the following strains: *Serratia plymuthica* strain A294, *Enterobacter amnigenus* strain A167, *Rahnella aquatilis* strain H145, *Serratia rubidaea* strain H440 and *Serratia rubidaea* strain H469, in essentially equal proportions - the composition according to the invention.
GT - a two-component mixture of protective bacteria containing the following strains: *Serratia plymuthica* strain A294 and *Serratia rubidaea* strain H440, in essentially equal proportions.

The invention has been described in the examples of the invention, the examples presented the comparison between the effect of use of the strains individually, in the mixture of the 2 strains and in the mixture of the 5 strains - the composition, and in the figures, where:

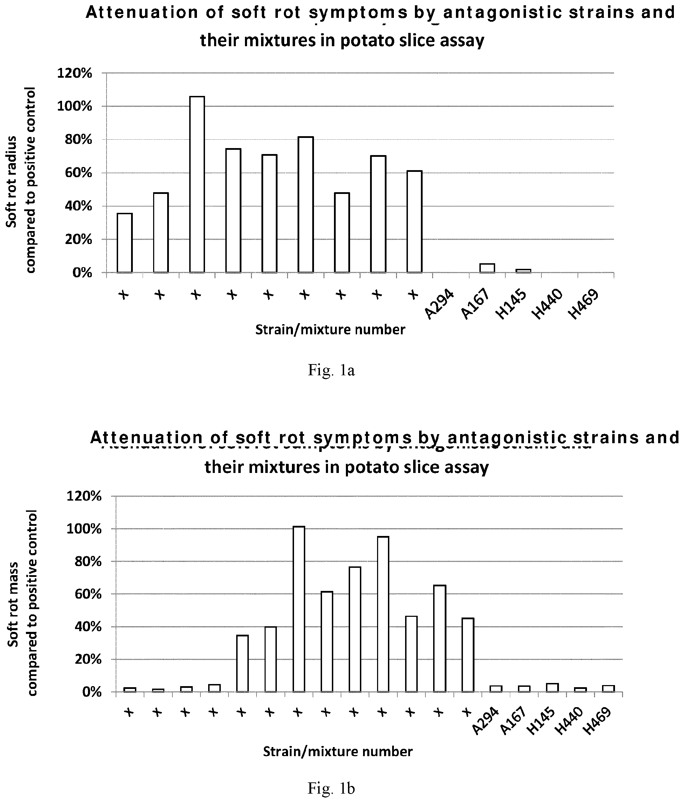

Fig. 1. - shows a selection of the 5 best antagonistic strains that protect potato tuber tissue against disease symptoms caused by pectinolytic bacteria;
   a) protective effect of the application of antagonistic bacteria as a % of the soft rot diameter relative to the control (reduction of the radius of soft rot of tubers, compared to the control containing only pathogenic bacteria from the genera *Pectobacterium* spp. and *Dickeya* spp.),
   b) protective effect of the application of antagonistic bacteria as a reduction of the mass of soft rot, compared to the control containing only pathogenic bacteria from the genera *Pectobacterium* spp. and *Dickeya* spp.
X - is an antagonist strain not disclosed in this patent application.

Selected strains: *Serratia plymuthica* strain A294, *Enterobacter amnigenus* strain A167, *Rahnella aquatilis* strain H145, *Serratia rubidaea* strains H440 and *Serratia rubidaea* strain H469 exhibited the best protective effect.

Fig. 2. - shows the bacterial growth of *Serratia plymuthica* strain A294, *Enterobacter amnigenus* strain A167, *Rahnella aquatilis* strain H145, *Serratia rubidaea* strains H440 and *Serratia rubidaea* strain H469 under 7°C.

Fig. 3. a-e - shows the bacterial growth of *Serratia plymuthica* strain A294, *Enterobacter amnigenus* strain A167, *Rahnella aquatilis* strain H145, *Serratia rubidaea* strains H440 and *Serratia rubidaea* strain H469 and their rifampicin-resistant mutants

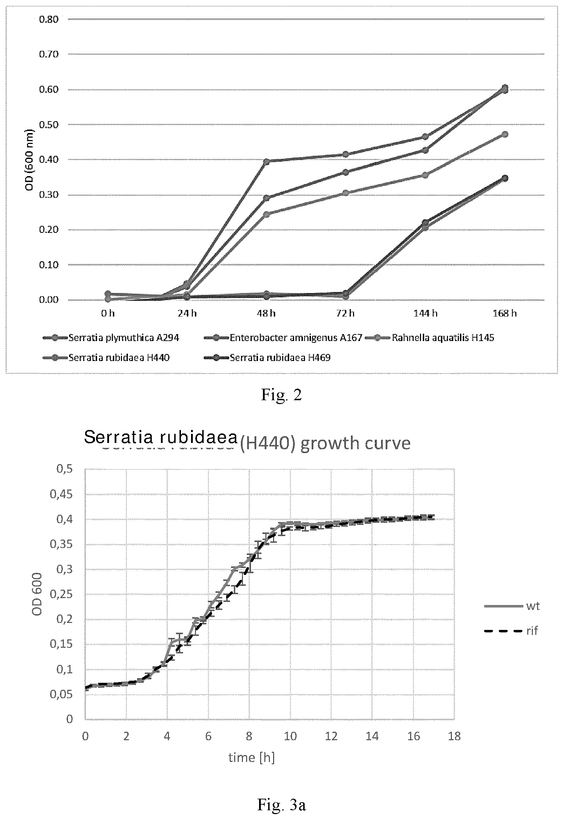

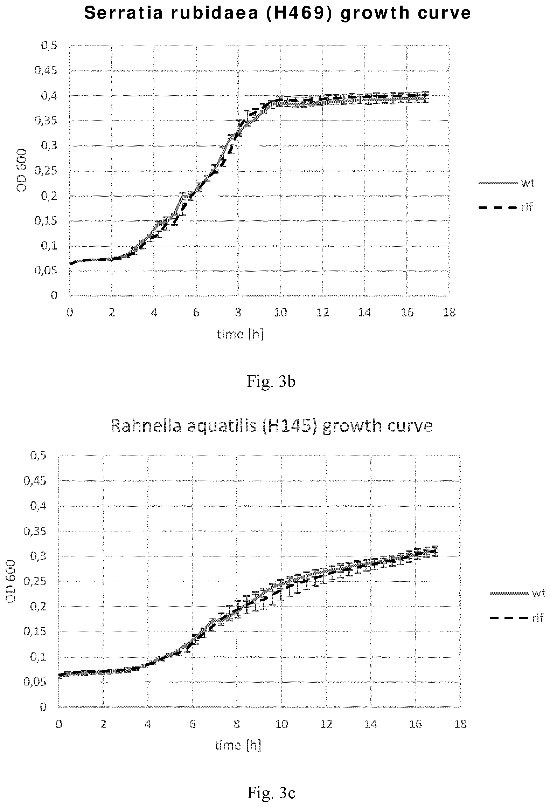

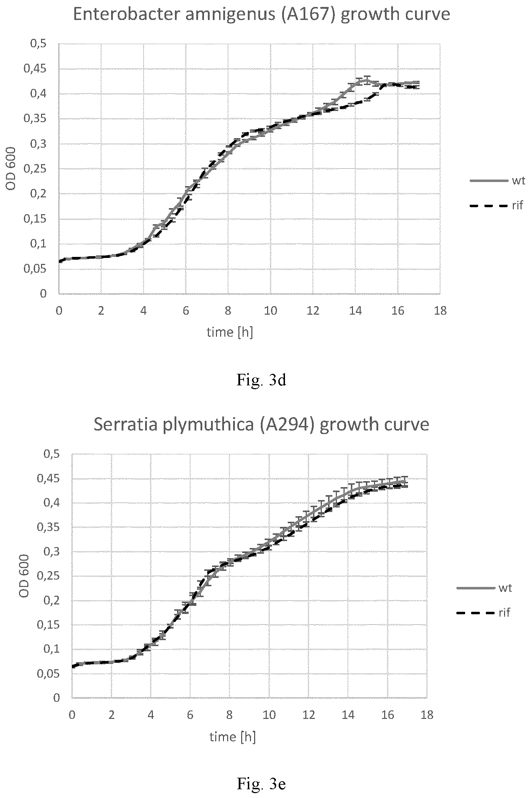

Legend to Fig. 3: wt - wild type strain, genetically unmodified, rif - genetically modified strain with resistance to antibiotic - rifampicin

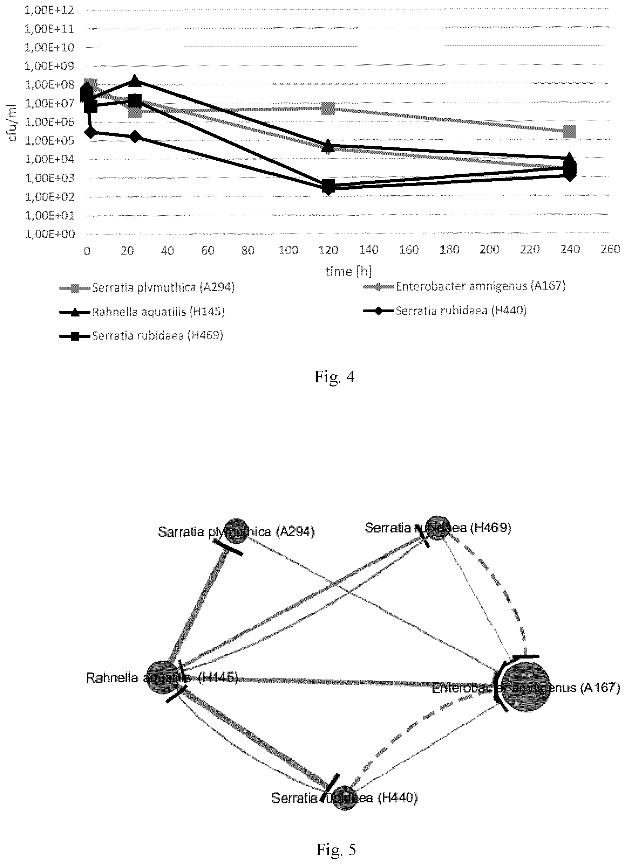

Fig. 4. - shows the survival of antagonistic strains in a mixture. Observations were carried out in PDB (potato dextrose broth) medium, pH 7.0, to which antagonistic bacteria were introduced to obtain an initial optical density of 3 on the McFarland scale. Bacterial growth over time is expressed in CFU / ml, and time in hours. The data averaged from three replications are presented.

Fig. 5. - shows the antagonistic interaction among the tested antagonistic strains in a mixture. The graph shows the ability of the tested strains to mutually inhibit the growth of one another on PDA medium (potato dextrose agar, pH=7,0). The thickness of the line is proportional to the radius of the growth inhibition zone. Dashed line is marking (not in scale) the production of a zone where the growth is visibly decreased but not fully inhibited. In the figure, the diameters of green circles for each strain are proportional to the number of strains inhibited by a given strain.

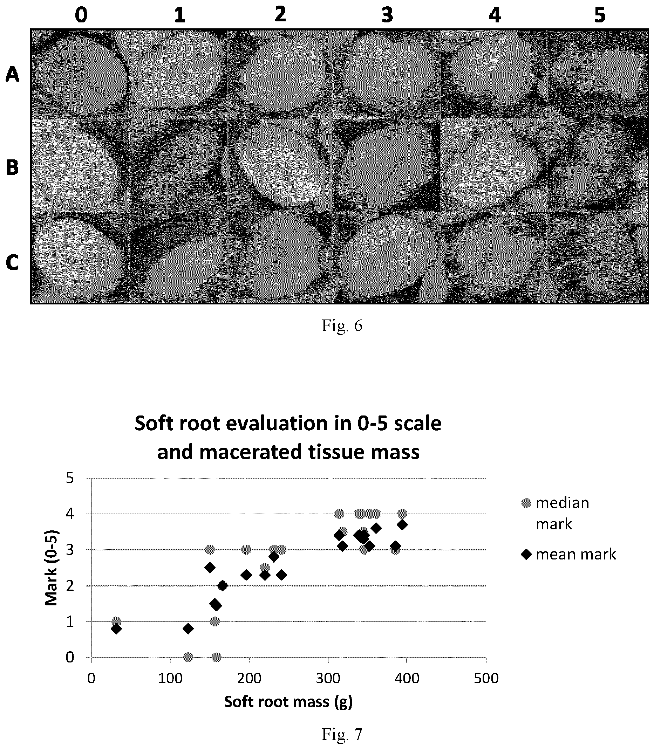

Fig. 6 - shows the scale of development of disease symptoms on potato tubers. Numbers 0-5 indicate the scale of development of disease symptoms, letters A-C - technical replications. Representative results are presented.

Fig. 7 - shows a comparison of the average mass of rotten potato tissue and the median rank of soft rot symptoms in the developed 6-point symptoms scale.

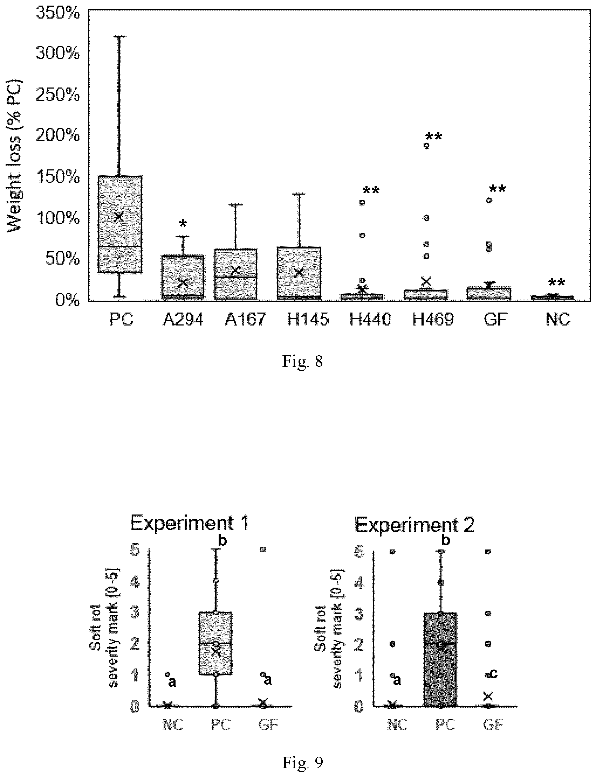

Fig. 8 - shows the protective effect of the presence of the antagonistic bacteria *Serratia plymuthica* strain A294, the *Enterobacter amnigenus* strain A167, the *Rahnella aquatilis* strain H145, the *Serratia rubidaea* strains H440 and the *Serratia rubidaea* strain H469.

Each strain was tested separately. Also tested was a mixture of 5 antagonist strains (GF) in the presence of pathogens. K (-) - negative control - uninoculated potato tubers, neither with protective nor pathogenic bacteria, K (+) - positive control, i.e. potato tubers inoculated only with pathogenic bacteria.

Fig. 9 - shows the assessment of the severity of soft rot symptoms on a six-point scale [0-5] for potato tubers inoculated with pathogens and pathogens in combination with a mixture of protective strains. The results obtained in two independent experiments (biological repetitions) are presented in separate graphs (Experiment 1 and Experiment 2). Symbols; NC: negative control (not inoculated with a mixture of pathogenic bacteria), PC: positive control (inoculated with water, followed by a mixture of pathogenic bacteria), GF: a five-component mixture of protective bacteria (inoculated with a mixture of protective bacteria and then with a mixture of pathogenic bacteria). Different letters above the bars indicate statistically significant differences between the data groups calculated using the Dunn's test (level of significance α = 0,05).

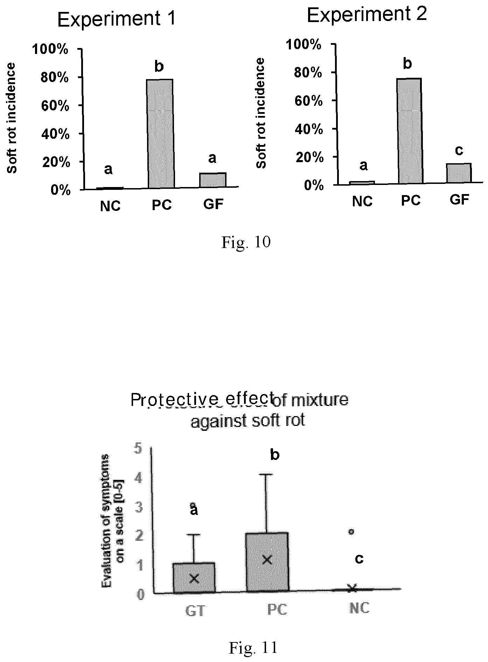

Fig. 10 - shows the percentage of potato tubers exhibiting the symptoms of soft rot (regardless of severity) in the sample. The results obtained in two independent experiments (biological repetitions) are presented in separate graphs (Experiment 1 and Experiment 2). Symbols; NC: negative control (not inoculated with a mixture of pathogenic bacteria), PC: positive control (inoculated with water, followed by a mixture of pathogenic bacteria), Gf: a mixture of protective bacteria (inoculated with a five-component mixture of protective bacteria and then with a mixture of pathogenic bacteria). Different letters above the bars indicate that there are statistically significant differences between the data groups calculated based on the Chi² test.

Fig. 11 - shows the assessment of the severity of soft rot symptoms on a six-point scale [0-5] for potato tubers inoculated with pathogens and pathogens in combination with a mixture of protective strains. The results of two biological replications are summarized in one graph. Symbols; NC: negative control (not inoculated with a mixture of pathogenic bacteria), PC: positive control (inoculated with water and then with a mixture of pathogenic bacteria), GT: a two-component mixture of protective bacteria (GT contains strains: *Serratia plymuthica* strain A294, and strain *Serratia rubidaea* H440 in essentially equal in proportions). Different letters above the bars indicate statistically significant differences calculated on the basis of Dunn's test α = 0,05.

The invention is illustrated by the following embodiments, not constituting its limitations. It should be understood, especially by a specialist, that, although the given examples according to the invention of the effect of the antagonistic bacterial strains on plants relate mainly to potato, the strains according to the invention can be used to protect other plant species against diseases caused by pectinolytic bacteria, in particular bacteria of the genera *Pectobacterium* and *Dickeya.* In the following examples, unless otherwise indicated, the manufacturers' instructions for specific materials and methods have been used.

### Example 1

### SELECTION OF ANTAGONISTIC BACTERIAL STRAINS FOR THE MIXTURE

The following were prepared: 15 mixtures containing antagonistic bacteria having various mechanisms of antagonistic effect against potato pathogens of the genera *Pectobacterium* and *Dickeya.* The mixtures were prepared in such a way that in each mixture there were up to 5 strains of antagonistic bacteria with different mechanism of antagonism against pectinolytic bacteria.

Based on the activity of the 15 mixtures (Table 1), through the comparative analysis of their composition, the strains (components of mixtures) were selected which provided a protective effect against pathogens of the genera *Pectobacterium* and *Dickeya,* and their effectiveness was confirmed in laboratory tests on potato slices (Fig 1); the strains were: *Serratia plymuthica* strain A294, *Enterobacter amnigenus* strain A167, *Rahnella aquatilis* strain H145, *Serratia rubidaea* strains H440 and *Serratia rubidaea* strain H469 (Table 1).

The mixture of antagonistic bacteria can be applied in a lyophilized form and / or in suspension.

Table 1. - shows the composition of the proposed 15 mixtures of antagonistic bacteria.

Legend: *Serratia plymuthica* strain A294, *Enterobacter amnigenus* strain A167, *Rahnella aquatilis* strain H145, *Serratia rubidaea* strains H440 and *Serratia rubidaea* strain H469, X - means other bacterial strains not disclosed in this patent application.

**Table 1.**

| Mixture | Component 1 | Component 2 | Component 3 | Component 4 | Component 5 |
|---|---|---|---|---|---|
| 1 | X | X | H440 | X | X |
| 2 | X | H145 | H469 | X | X |
| 3 | X | X | A294 | X | X |
| 4 | X | X | H440 | A167 | X |
| 5 | X | A294 | X | X | A167 |
| 6 | X | X | H145 | X | X |
| 7 | X | X | X | X | H440 |
| 8 | X | X | X | X | X |
| 9 | X | H145 | X | X | X |
| 10 | X | X | X | X | X |
| 11 | X | X | X | X | X |
| 12 | A167 | X | H469 | X | X |
| 13 | H440 | X | X | X | X |
| 14 | X | X | A294 | H145 | H469 |
| 15 | X | X | X | X | X |

### Example 2

### CONFIRMATION OF THE SPECIES AFFILIATION OF THE SELECTED ANTAGONISTIC BACTERIA

In order to determine in detail: the genus and species of selected antagonistic strains, identification was performed based on the sequence similarity analysis of the coding 16S rRNA gene to sequences for other microorganisms, available in public databases (e.g., GenBank). Amplification of a very long (> 1400 nucleotides) fragment of the 16S rRNA gene was performed, thus enabling precise identification of the species of the tested strains. The results of species identification obtained on the basis of sequencing of the almost complete 16S rRNA gene are presented in Table 2.

Table 2. - shows the antagonistic strains with the greatest protective potential and their species affiliation, determined on the basis of phylogenetic analysis of the 16S rRNA gene sequence of approximately 1,500 nucleotides.

**Table 2.**

| Strain | Species determined on the basis of the complete 16S rRNA sequence |
|---|---|
| A294 | *Serratia plymuthica* |
| A167 | *Enterobacter amnigenus* |
| H145 | *Rahnella aquatilis* |
| H440 | *Serratia rubidaea* |
| H469 | *Serratia rubidaea* |

All selected strains were identified to the species level.

### Example 3

### DETERMINATION OF ANTAGONIST BACTERIA CAPACITY FOR GROWTH IN TEMPERATURE OF STORAGE OF TUBERS (7 °C) AND OTHER TEMPERATURES (10°C, 28°C and 40 °C)

Due to the fact that an effective mixture of antagonistic bacteria should work in a wide range of temperatures that may occur when using the mixture on cultivated plants, it has become important to determine the ability of microorganisms potentially constituting components of the protective formulation for survival and growth at different temperatures. During the tests, the following was determined: the ability of the tested antagonist strains to grow at 7 °C, 10 °C, 28 °C and 40 °C, on solid and liquid media. The following are the results for five strains selected on the basis of experiments with potato tubers.

The culture at 7 ° C was carried out in TSB medium (Oxoid) (liquid medium) in 24-well plates (Falcon) with shaking. The medium was inoculated with the test bacteria by adding overnight cultures (50: 1, volume/volume, v/v). As a control, the following was used: the psychrotolerant strain *Bacillus weihenstephanensis* CCM 4872 (=DSM 11821^{T}). Six technical replicates were performed for each of the tested strains. Measurement of bacterial cell density (λ=600 nm) was carried out every 24 h for 7 days using a 1420 Multiple Counter Victor (Wallac) plate reader (Fig. 2).

The culture on a solid medium was carried out at 7 °C, 10 °C, 28 °C and 40 °C on TSA medium (Oxoid) in 24-well plates (800µl medium / well). Two µl of the bacterial suspension, prepared by dilution (50: 1, v / v) of an overnight culture of the tested strains, was spotted onto each of the "microplates". As a control, the following was used: the psychrotolerant strain *B. weihenstephanensis* CCM 4872 (=DSM 11821T). Two technical replicates were made for each temperature tested. The bacterial growth was evaluated every 24 hours, on a binominal scale: "+" - growth, "-" - no growth). The observation was carried out for 7 days (Table 3).

Table 3 - shows the growth of the bacteria *Serratia plymuthica* strain A294, the *Enterobacter amnigenus* strain A167, the *Rahnella aquatilis* strain H145, the *Serratia rubidaea* strain H440 and the *Serratia rubidaea* strain H469 at the following temperatures: 7°C, 10°C, 28°C and 40 °C.

**Table 3**

| Growth temperature | Culture time | A294 | A167 | H145 | H440 | H469 |
|---|---|---|---|---|---|---|
| 7 °C | 24 h | - | - | - | - | - |
| | 48 h | - | - | - | - | - |
| | 72 h | + | + | + | + | + |
| | 96 h | + | + | + | + | + |
| 10 °C | 24 h | - | - | - | - | - |
| | 48 h | + | + | + | - | - |
| | 72 h | + | + | + | + | + |
| | 96 h | + | + | + | + | + |
| 28 °C | 24 h | + | + | + | + | + |
| | 48 h | + | + | + | + | + |
| | 72 h | + | + | + | + | + |
| | 96 h | + | + | + | + | + |
| 40 °C | 24 h | + | + | + | + | + |
| | 48 h | + | + | + | + | + |
| | 72 h | + | + | + | + | + |
| | 96 h | + | + | + | + | + |
| Legend | | | | | | |
| + | Growth observed | | | | | |
| - | No growth observed | | | | | |

Among the selected five antagonist strains, all are able to grow in the range of temperatures tested, i.e. at 7°C, 10°C, 28°C and 40 °C.

### Example 4

### SURVIVAL RATE OF ANTAGONISTIC BACTERIAL STRAINS IN THE MIXTURE OF ANTAGONISTIC BACTERIA

To assess the survival rate of the antagonistic bacterial strains in the protective mixture, what was obtained, by selection of spontaneous mutants (seeding on the antibiotic medium and passage of bacterial cultures in the presence of antibiotics), were rifampicin-resistant mutants of the bacteria *Serratia plymuthica* strain A294, the *Enterobacter amnigenus* strain A167, the *Rahnella aquatilis* strain H145, the *Serratia rubidaea* strain H440 and the *Serratia rubidaea* strain H469. The growth of the rifampicin-resistant mutants was then compared with the growth of wild-type (non-mutated) strains to determine whether antibiotic-resistant mutants exhibit similar generation time.

In order to determine whether the obtained mutants exhibit the same growth rate, growth curves were made for them and they were compared with the growth curves for the wild-type strains (Fig. 3).

Then, using the acquired rifampicin-resistant (antibiotic-resistant) mutants, separately for each mutant of the antagonist strain, the survival was tested in a mixture of four other wild-type antagonist strains. Each of the bacterial mixtures was made by adding, in equal proportions, individual strains with a specific optical density (3 McF). In each mixture, one strain was labelled (resistant to rifampicin), the others were not. The mixtures thus prepared were grown with shaking in liquid cultures (PDB - potato dextrose broth) (liquid medium) and cultured on a medium with rifampicin to count viable bacterial cells (Fig. 4). All strains of antagonistic bacteria survived in a mixture containing other antagonistic strains, which means that the antagonistic bacteria strains in the mixture do not limit one another's growth.

### Example 5

### DETERMINATION OF POTENTIAL MUTUAL ANTAGONISM OF ANTAGONISTIC STRAINS

Antagonistic strains may not only inhibit the growth of pathogenic strains of the genera *Pectobacterium* and *Dickeya* (desirable effect), but also negatively affect the other antagonistic strains present in the mixture / protective formulation (negative effect). Therefore, it was reasonable to analyse the growth inhibition potential of antagonistic strains by other protective strains. As part of the experiments, mutual antagonism was tested between the following strains: *Serratia plymuthica* strain A294, *Enterobacter amnigenus* strain A167, *Rahnella aquatilis* strain H145, *Serratia rubidaea* strains H440 and *Serratia rubidaea* strain H469.

The plates with PDA medium (solid medium, containing the potato extract) were inoculated, by spreading the bacterial suspension on the surface of the medium, with one of the pools of tested protective strains. Subsequently, 2µl of the remaining strains were applied to the pre-inoculated plate.

The greatest ability to inhibit the growth of four other antagonist strains tested, is demonstrated by: the *Rahnella aquatilis* strain H145. In contrast, the inability to inhibit the growth of other antagonistic strains is exhibited by the *Enterobacter amnigenus* strain A167 (Fig. 5).

### Example 6

### PROTECTIVE EFFECT OF APPLICATION OF ANTAGONISTIC STRAINS AND THEIR MIXTURE AGAINST BACTERIA OF THE GENERA PECTOBACTERIUM AND DICKEYA ON WHOLE (NOT WOUNDED) POTATO TUBERS

The potential of 5 protective strains was analysed: the *Serratia plymuthica* strain A294, the *Enterobacter amnigenus* strain A167, the *Rahnella aquatilis* strain H145, the *Serratia rubidaea* strains H440 and the *Serratia rubidaea* strain H469, to limit the development of disease symptoms caused by bacteria of the genera *Pectobacterium* and *Dickeya.* Each of the protective isolates was tested both individually and in a mixture with the other strains (a mixture of 5 antagonistic strains was designated as GF) on potato tubers in the presence of pathogens.

The GF mixture - the composition according to the invention - contains the following strains: the *Serratia plymuthica* strain A294, the *Enterobacter amnigenus* strain A167, the *Rahnella aquatilis* strain H145, the *Serratia rubidaea* strains H440 and the *Serratia rubidaea* strain H469 in substantially equal proportions of cell numbers.

For this purpose, a detailed protocol has been developed, as follows:
- 24 hours before the planned experiment, potato tubers should be transferred from cold storage conditions to room temperature (around 20 - 22°C) to equalize the temperature in tubers.
- *Preparation of potato tubers for the test.* Potatoes should be washed in tap water, surface-sterilized by incubating for 20 min in 5% sodium hypochlorite solution, rinsed twice in tap water and dried in air in a laminar chamber (time: 10-15 minutes).
- *Preparation of suspensions of antagonistic bacteria.* Suspensions of antagonistic bacteria (each separately) should be prepared in tap water (carrier) until a homogeneous mixture is obtained to give a final concentration of 10⁸ CFU / ml (colony forming units / ml). When testing a single protective strain, the turbidity of the suspension should be 3 units on the McFarland (McF) scale. When testing a mixture of strains, the turbidity should be "n" x 3 McF, where "n" is the number of strains in the target mixture (assuming a mixture that contains 5 antagonist strains, n = 5, assuming a mixture that contains 4 antagonist strains, n = 4, etc.). When testing mixtures, all components (strains) to be poured off in the same volume / volume ratio (v: v) just before starting the experiment.
- *Preparation of mixtures of antagonistic bacteria.* A mixture of antagonists (protective bacteria, antagonistic bacteria) is prepared just before the start of the experiment by blending all components of the mixture (antagonist bacteria) in equal volume / volume ratios (v / v). The mixture of antagonistic bacteria can be applied in a lyophilized form and / or in suspension.
- *Preparation of suspensions of pathogenic strains.* Suspensions of pectinolytic bacteria (pathogenic bacteria) to be prepared, for each strain individually, in water until a homogeneous consistency of the mixture is achieved to give a final concentration of 10⁶ CFU / ml (colony forming units / ml). For this purpose, the density of the mixture to be measured in a densitometer and brought to a value of 0.03 on the McF scale. Suggested preparation of suspensions 10 times concentrated 0.3 McF and dilution to the required concentration. Pathogen suspensions should be mixed immediately before infiltration (in the same volume / volume ratio (v/v)).

Two methods were tested in which this was done:
1. inoculations of potato tubers with pathogenic strains followed by protective strains (antagonistic bacteria)
2. inoculations of potato tubers with protective strains (antagonistic bacteria) followed by pathogenic strains

The second method proved to be more effective and therefore it is preferred, however both are possible to be used on a larger scale (e.g. semi-technical, technical, etc.). The presented protocol has been developed for a laboratory experimental scale, but the scale of the experiment can be arbitrarily increased, if necessary, by increasing the number of potato tubers and the volume and concentration of individual components and populations of antagonistic bacteria):
Inoculation of tubers - mixtures of protective strains (antagonistic bacteria). Potatoes, in packs of 10, to be put in a plastic, 2-litre beaker and poured over with prepared suspensions of protective bacteria (antagonistic bacteria) (about 1.2 litres of a mixture necessary per strain to cover the potatoes in a beaker) or with tap water for control samples. Beakers to be placed in a desiccator with a diameter of 60 cm. Close the desiccator and turn on the vacuum pump. After reaching the vacuum of -65 bar, set the timer to 10 minutes. During this time, the negative pressure will increase to approximately -80 bar and will be maintained until the end of incubation. Then, open the desiccator valve (level the vacuum) and wait 10 minutes without removing the potatoes from the bacterial suspension. Then, remove the potatoes from the suspension, place in plastic, openwork containers (baskets) (dimensions e.g. 35 x 25 x 14 cm) and leave to dry at room temperature (20°C ± 2°C) for 16 -24 hours (in the open air).

Inoculation of tubers - mixtures of pathogenic strains (pectinolytic bacteria). Potatoes (previously inoculated with a suspension of protective strains (antagonistic bacteria) or uninoculated), in packs of 10, to be put in plastic beakers, poured over with the prepared suspension of pathogenic strains and placed in the desiccator. Place the beakers in a desiccator with a diameter of 60 cm. Close the desiccator and switch on the vacuum pump. After reaching the vacuum of -65 bar, set the timer to 10 minutes. During this time the negative pressure is around -80 bar. Then open the desiccator valve (level the vacuum) and wait 10 minutes without removing the potatoes from the bacterial suspension.

Preparation of tubers after inoculation and incubation of tubers to determine the scale of disease symptoms. Remove the infected tubers from the beakers and leave to dry in the open air for 70 minutes after infection. Place 10 tubers in plastic boxes which contain metal grids that prevent direct contact of the inoculated tubers with the bottom of the box. Placed at the bottom there is lignin flooded with 130 ml of distilled water (creating conditions of increased humidity - relative humidity of about 85-90%). Prepared tubers should be incubated for 5 days at 28°C in closed containers, and after that time, the symptoms of disease should be evaluated on each infected tuber (evaluation of symptoms on a 6-point scale, developed for the test, is below).

Description of a 6-stage scale of evaluation of the development of disease symptoms on potato tubers, used to determine the protective effect of protective preparations:
Evaluation 0 - no disease symptoms whatsoever on the tubers
Evaluation 1 - soft rot on the surface in one or more points, but with a limited area (total less than 25% of the total surface of the tuber).
Evaluation 2 - symptoms as in evaluation 1, but the area of rotting is 25% -50% of the surface of the tuber
Evaluation 3 - symptoms as in evaluation 1 and 2, but additionally there is a delamination of the tuber skin, the area of deterioration 50% -90% of the total surface of the tuber
Evaluation 4 - symptoms as in evaluation 3, area of rotting over 90% of the total surface of the tuber, tuber core non-macerated
Evaluation 5 - tuber completely macerated (100% maceration of tuber tissues) Photographic imagery illustrating the severity of the disease symptoms described above is shown in Fig. 6.

For the purpose of evaluating the adopted scale of disease symptoms on potato tubers, the average and median of the scores obtained for a given sample were compared with the mass of soft rot obtained for the same sample. The mass of soft rot was obtained by comparing the initial mass of potatoes in a given sample with the mass remaining after rinsing the macerated tissue (Fig. 7). The obtained results showed that the evaluation in the proposed 6-point scale is a good measure of the development of soft rot symptoms, and due to the lower labour intensity of the evaluation in the proposed scale with respect to the need to weigh rotten tissue, it is a better solution for larger-scale experiments.

Under the proposed experimental conditions, all antagonistic strains were able to limit, in a statistically significant way, the development of disease symptoms on potato tubers caused by bacteria of the genera *Pectobacterium* and *Dickeya* (Fig. 8).

### Example 7

### PROTECTIVE EFFECT OF APPLICATION OF A FIVE-COMPONENT MIXTURE OF ANTAGONISTIC BACTERIA AGAINST BACTERIA OF THE GENERA PECTOBACTERIUM AND DICKEYA ON WHOLE (NOT WOUNDED) POTATO TUBERS AFTER THE PERIOD OF COLD STORAGE

Potato tubers (seed potatoes of Irga variety) were infiltrated with a mixture of protective strains and a mixture of pathogenic strains according to the protocol in Example 6 in version 2, with the difference that potatoes were infiltrated, in packs of 30, in 5 litre beakers flooded with a 2-litre mixture. In one experiment, 5 technical repetitions (5 separate infiltrations) were carried out for each of the tests. Infiltrated potatoes were allowed to dry and then placed in plastic boxes in packs of 30. Closed boxes were placed in a cold room (at 8 °C, humidity 80%) for a period of six months. After 6 months of incubation, the potatoes were transferred to challenge conditions for symptoms of soft rot (temperature 28 °C, humidity 90%). Tubers, in packs of 15, were placed in plastic boxes and incubated for 5 days at 28 °C in closed containers, and after that time, evaluation of disease symptoms on each infected tuber (evaluation of symptoms on a 6-point scale, developed for the test; description is given in example 6). The experiment was performed in two independent repetitions. In total, each combination was tested on 300 tubers. Symptom evaluations were compared between the control, an inoculated mixture of pathogenic bacteria and the inoculated mixture of protective bacteria samples and then a mixture of pathogenic bacteria. The results were compared in terms of intensity of symptoms and frequency of occurrence.

In both experiments there was a significant difference (Kruskal-Wallis 1: chi² = 204, df = 2, p < 2,2e- 16; 2: chi ²= 211,93, df = 2, p = 2.2e-16) between evaluations of potatoes infiltrated with a mixture of protective bacteria and the unprotected potatoes (Fig. 9). GF mixture exhibited a statistically significant efficacy and decreased the average score of symptoms by 83-94%.

The results look similar if we analyse the share of potatoes showing symptoms of soft rot (regardless of the severity) (Fig. 10). There is a significant difference between the number of rotten potatoes in the positive control and the samples infiltrated with the mixture of protective bacteria, (1: Chi² = 131,44, df = 1, p = 1,98e-30; 2: Chi² = 112,80, df = 1, p = 4,28e-143). The frequency of symptoms of soft rot is reduced by a mixture of protective strains by 82-96%. The effectiveness of the strains was also pre-confirmed individually.

### Example 8

### PROTECTIVE EFFECT OF APPLICATION OF TWO-COMPONENT MIXTURE OF ANTAGONISTIC STRAINS AGAINST BACTERIA OF THE GENERA PECTOBACTERIUM AND DICKEYA ON WHOLE (NOT WOUNDED) POTATO TUBERS

Potatoes were infiltrated with a mixture of protective (antagonist) strains and a mixture of pathogenic strains according to the protocol in Example 6, with the difference that a two-component mixture was tested. (GT; contains strains: *Serratia plymuthica* strain A294, and *Serratia rubidaea* strain H469 in substantially equal proportions). Evaluation of the symptoms was performed as described in Example 6.

In the experimental conditions proposed, the two-component mixture also showed a protective effect (Kruskal-Wallis chi²=24,422, df=2, p=4,975e-06, Dunn test GT Z = - 2.766769 p = 1,132296e-02;) to reduce the development of disease symptoms on potato tubers caused by bacteria of the genera *Pectobacterium* and *Dickeya* (Fig. 11).

## Claims

1. Composition of antagonistic bacterial strains for protection of plants against bacterial infections or treatment of plants infected with bacteria, **characterized in that** the composition comprises five strains:
- strain of the antagonistic bacteria *Serratia plymuthica* A294 being deposited on 1 December 2017 with the Polish Collection of Microorganisms PCM at the Ludwik Hirszfeld Institute of Immunology and Experimental Therapy in Wroclaw, Polish Academy of Sciences, under number B/00143, and
- strain of the antagonistic bacteria *Serratia rubidaea* H469 being deposited on 1 December 2017 with the Polish Collection of Microorganisms PCM at the Ludwik Hirszfeld Institute of Immunology and Experimental Therapy in Wroclaw, Polish Academy of Sciences, under number B/00142, and
- strain of the antagonistic bacteria *Serratia rubidaea* H440 being deposited on 1 December 2017 with the Polish Collection of Microorganisms PCM at the Ludwik Hirszfeld Institute of Immunology and Experimental Therapy in Wroclaw, Polish Academy of Sciences, under number B/00141, and
- strain of the antagonistic bacteria *Enterobacter amnigenus* A167 being deposited on 1 December 2017 with the Polish Collection of Microorganisms PCM at the Ludwik Hirszfeld Institute of Immunology and Experimental Therapy in Wroclaw, Polish Academy of Sciences, under number B/00145, and
- strain of the antagonistic bacteria *Rahnella aquatilis* H145 being deposited on 1 December 2017 with the Polish Collection of Microorganisms PCM at the Ludwik Hirszfeld Institute of Immunology and Experimental Therapy in Wroclaw, Polish Academy of Sciences under number B/00144.

2. Composition according to the claim 1, wherein all strains in the composition are in equal number relative to one another.

3. Composition according to the claims 1 or 2, wherein the number of cells of each strain is at least 10¹ cells per millilitre CFU / ml, preferably 10⁸ CFU / ml.

4. Composition according to one of the claims 1-3, wherein the composition is in a lyophilized form or in a suspension.

5. Composition according to one of the claims 1-4, wherein the strains are metabolically active.

6. Use of the composition described in the claim 1 or the claim 2 or the claim 3 or the claim 4 or the claim 5, for prevention or treatment of potato infections caused by pectinolytic bacteria *Dickeya* spp. and/or *Pectobacterium* spp.

## Patentansprüche

1. Zusammensetzung von antagonistischen Bakterienstämmen zum Schutz von Pflanzen gegen bakterielle Infektionen oder zur Behandlung von mit Bakterien befallenen Pflanzen, **dadurch gekennzeichnet, dass** die Zusammensetzung fünf Stämme umfasst:
- Stamm des antagonistischen Bakteriums *Serratia Plymuthica* A294, erfasst am 1. Dezember 2017 bei der Polnischen Sammlung von Mikroorganismen PCM am Ludwik-Hirszfeld-Institut für Immunologie und experimentelle Therapie in Breslau, Polnische Akademie der Wissenschaften, unter der Nummer B/00143, und
- Stamm des antagonistischen Bakteriums *Serratia Rubidaea* H469, erfasst am 1. Dezember 2017 bei der Polnischen Sammlung von Mikroorganismen PCM am Ludwik-Hirszfeld-Institut für Immunologie und experimentelle Therapie in Breslau, Polnische Akademie der Wissenschaften, unter der Nummer B/00142, und
- Stamm des antagonistischen Bakteriums *Serratia Rubidaea* H440, erfasst am 1. Dezember 2017 bei der Polnischen Sammlung von Mikroorganismen PCM am Ludwik-Hirszfeld-Institut für Immunologie und experimentelle Therapie in Breslau, Polnische Akademie der Wissenschaften, unter der Nummer B/00141, und
- Stamm des antagonistischen Bakteriums *Enterobacter Amnigenus* A167, erfasst am 1. Dezember 2017 bei der Polnischen Sammlung von Mikroorganismen PCM am Ludwik-Hirszfeld-Institut für Immunologie und experimentelle Therapie in Breslau, Polnische Akademie der Wissenschaften, unter der Nummer B/00145, und
- Stamm des antagonistischen Bakteriums *Rahnella aquatilis* H145, erfasst am 1. Dezember 2017 bei der Polnischen Sammlung von Mikroorganismen PCM am Ludwik-Hirszfeld-Institut für Immunologie und experimentelle Therapie in Breslau, Polnische Akademie der Wissenschaften, unter der Nummer B/00144.

2. Die Zusammensetzung nach Anspruch 1 **dadurch gekennzeichnet** alle Stämme in der Zusammensetzung in gleicher Anzahl zueinander vorliegen.

3. Die Zusammensetzung nach Ansprüchen 1 oder 2**dadurch gekennzeichnet** die Anzahl der Zellen jedes Stammes mindestens 10¹ Zellen pro Milliliter KBE/ ml, vorzugsweise 10⁸ KBE / ml, beträgt.

4. Die Zusammensetzung nach einem der Ansprüche 1 bis 3 **dadurch gekennzeichnet** die Zusammensetzung in einer lyophilisierten Form oder in einer Suspension vorliegt.

5. Die Zusammensetzung nach einem der Ansprüche 1-4**dadurch gekennzeichnet** die Stämme metabolisch aktiv sind.

6. Verwendung Die Zusammensetzung im Anspruch 1 oder Anspruch 2 oder Anspruch 3 oder Anspruch 4 oder Anspruch 5 beschriebenen Zusammensetzung zur Vorbeugung oder zur Behandlung von Kartoffelinfektionen, die durch pektinolytische Bakterien *Dickeya* spp. und/oder *Pectobacterium* spp. hervorgerufen werden.

## Revendications

1. Composition de souches bactériennes antagonistes pour la protection des plantes contre les infections bactériennes ou le traitement des plantes infectées par des bactéries, qui se **caractérise par le fait qu'**elle comprend cinq souches :
- souche de la bactérie antagoniste *Serratia plymuthica* A294 déposée le 1er décembre 2017 auprès de la Collection polonaise de micro-organismes PCM de l'Institut d'immunologie et de thérapie expérimentale Ludwik Hirszfeld à Wroclaw, Académie polonaise des sciences, sous le numéro B/00143, et
- souche de la bactérie antagoniste *Serratia rubidaea* H469 déposée le 1er décembre 2017 auprès de la Collection polonaise de micro-organismes PCM à l'Institut d'immunologie et de thérapie expérimentale Ludwik Hirszfeld de Wroclaw, Académie polonaise des sciences, sous le numéro B/00142, et
- souche de la bactérie antagoniste *Serratia rubidaea* H440 déposée le 1er décembre 2017 auprès de la Collection polonaise de micro-organismes PCM de l'Institut d'immunologie et de thérapie expérimentale Ludwik Hirszfeld à Wroclaw, Académie polonaise des sciences, sous le numéro B/00141, et
- souche de la bactérie antagoniste *Enterobacter amnigenus* A167 déposée le 1er décembre 2017 auprès de la Collection polonaise de micro-organismes PCM à l'Institut d'immunologie et de thérapie expérimentale Ludwik Hirszfeld de Wroclaw, Académie polonaise des sciences, sous le numéro B/00145, et
- souche de la bactérie antagoniste *Rahnella aquatilis* H145 déposée le 1er décembre 2017 auprès de la Collection polonaise de micro-organismes PCM de l'Institut d'immunologie et de thérapie expérimentale Ludwik Hirszfeld à Wroclaw, Académie polonaise des sciences, sous le numéro B/00144.

2. Composition selon la revendication 1 est **caractérisée en ce que** toutes les souches sont en nombre égal.

3. Composition selon les revendications 1 ou 2, est **caractérisée en ce qu'**e le nombre de cellules de chaque souche est d'au moins 10¹ cellules par millilitre CFU / ml, de préférence 10⁸ CFU / ml.

4. Composition selon l'une des revendications 1 à 3, est **caractérisée en ce que** la composition est sous une forme lyophilisée ou en suspension.

5. Composition selon l'une des revendications 1 à 4, est **caractérisée en ce que** les souches sont métaboliquement actives.

6. Utilisation de la composition défini dans les revendication 1, la revendication 2, la revendication 3, la revendication 4 ou la revendication 5, pour la prévention ou le traitement des infections de la pomme de terre causées par les bactéries pectinolytiques *Dickeya* spp. et/ou *Pectobacterium* spp.
